# EUROPÄISCHE PATENTANMELDUNG

(11) **EP 0 960 633 A2**
(43) Veröffentlichungstag der Anmeldung: **01.12.1999**
(21) Anmeldenummer: 99250168.4
(22) Anmeldetag: 27.05.1999
(51) Int. Cl.: A61N 5/10

(54) **Radioaktive Röntgenquelle und deren Verwendung zur intrakavitären und intravaskulären Gewebebestrahlung**

(30) Priorität: 27.05.1998 DE 19825397
(71) Anmelder: Eurotope Entwicklungsgesellschaft für Isotopentechnologien mbH, 13125 Berlin (DE)
(72) Erfinder: Jürgen Ziegler, 12679 Berlin (DE)
(74) Vertreter: Ziebig, Marlene, Dr. Dipl.-Chem.

(57) **Zusammenfassung**

Die Erfindung betrifft eine an - und abschaltbare radioaktive Röntgenquelle, die eine β-Strahlung emittierende radioaktive Strahlenquelle (4) und einen Strahlungswandler (2) beinhaltet, wobei sie dadurch gekennzeichnet ist, daß sie ein Führungsrohr (3) zum axialen Verschieben der darin angeordneten radioaktiven Strahlenquelle (4) mittels eines Bewegungselementes (5), das an der Strahlenquelle (4) vorhanden ist, aufweist, daß der Teil des Führungsrohres (3), der an den distalen Führungskopf (1) des Führungsrohres (3) angrenzt, als Strahlungswandler (2) ausgestaltet ist, und daß der proximal an den Strahlungswandler (2) angrenzende Teil des Führungsrohres (3) aus einem die β-Strahlung absorbierenden Material besteht.

## Beschreibung

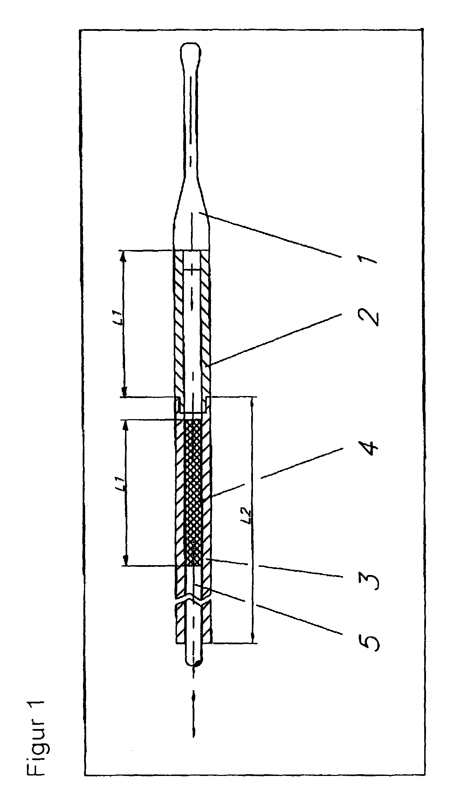

Die Erfindung betrifft eine an - und abschaltbare radioaktive Röntgenquelle, die eine β-Strahlung emittierende radioaktive Strahlenquelle und einen Strahlungswandler beinhaltet. Das Nuklid der radioaktiven Quelle kann beispielsweise Promethium (Pm)-147, Strontium (Sr)-89, Thallium (Tl)-204 oder Thulium (Tm)-170 sein. Der Strahlungswandler kann zum Beispiel aus Zink, Barium, Tellur, Wolfram oder Silber bestehen. Die erfindungsgemäße radioaktive Röntgenquelle emittiert im wesentlichen weiche Röntgenstrahlung und kann in miniaturisierter Form zur intrakavitären und intravaskulären Gewebebestrahlung eingesetzt werden, insbesondere zur Brachytherapie und Restenoseprävention. Figur 1 zeigt die erfindungsgemäße Röntgenquelle in einer vorteilhaften Ausgestaltung.

In der Medizin werden zur Strahlentherapie in Abhängigkeit vom zu behandelnden Gewebe und dessen Zugänglichkeit in Form und Strahlungsart unterschiedlichste Strahlenquellen eingesetzt. So kommen beispielsweise in der Brachytherapie Strahlenquellen oder radioaktive Stents zur Anwendung, die β-Strahlung, weiche Röntgenstrahlung oder auch eine Kombination aus β- und γ-Strahlung als wirksame Strahlungsart emittieren. In der onkologischen Praxis werden gegenwärtig zur Bestrahlung von Tumorgewebe die Radionuklide Iridium-192, Caesium-137, Cobalt-60 als β-/γ-Strahler unter überwiegender Nutzung der γ-Strahlung eingesetzt.

Es wird für die oben genannten medizinischen Zwecke in der Regel nach Strahlenquellen gesucht, die eine optimale Halbwertszeit besitzen und lange benutzbar sind. Die Strahlung sollte das gesunde Gewebe in der Umgebung des zu bestrahlenden Defektes/Tumors wenig oder nicht schädigen. Die Dosisleistung des Strahlers sollte eine Applikation von 15-20 Gray in 3 min. in 2 mm Abstand im Gewebe ermöglichen. Besonders wünschenswert sind solche Strahlenquellen, die sich je nach den medizinischen Gegebenheiten "an- oder abschalten" lassen, ohne sie für jeden Fall aus dem Körper entfernen und wieder einführen zu müssen, da dabei eine unbeabsichtigte Bestrahlung gesunden Gewebes nicht ausgeschlossen werden kann, und das Einführen und Entfernen stets eine Belastung für den Patienten bedeutet.

Es wurde nun eine radioaktive Röntgenquelle entwickelt, die aus einer β-Strahlung emittierenden radioaktiven Strahlenquelle (4) und einem Strahlungswandler (2) besteht, der die emittierte β-Strahlung in Röntgenstrahlung umwandelt. Das Nuklid der radioaktiven Quelle (4) ist vorzugsweise ausgewählt aus Promethium (Pm)-147, Strontium (Sr)-89, Thallium (Tl)-204 oder Thulium (Tm)-170. Besonders bevorzugt wird Promethium (Pm)-147 eingesetzt. Der die β-Strahlung in Röntgenstrahlung umwandelnde Strahlungswandler (2) kann vorzugsweise aus Zink, Barium, Tellur, Wolfram oder Silber bestehen. Besonders bevorzugt wird Silber eingesetzt. Die Fig. 1 zeigt die erfindungsgemäße Röntgenquelle in einer vorteilhaften Ausgestaltung. Die Röntgenquelle beinhaltet ein Führungsrohr (3) mit einem distalen Führungskopf (1), der zum Einführen der Quelle in eine Körperkavität oder in einen Katheder optimal ausgestaltet ist. Innerhalb des Führungsrohres (3) ist die radioaktive Quelle (4) mittels eines Bewegungselementes (5) axial verschiebbar, so daß die radioaktive Quelle (4) in den Teil des Führungsrohres (3), der in dieser Ausführungsform den Strahlungswandler (2) darstellt, hinein- und herausbewegt werden kann. Damit ist ein "An- und Abschalten" der erfindungsgemäßen Röntgenquelle möglich. Dazu muß der Strahlungswandler (2) mindestens die Länge der radioaktiven Strahlenquelle (4) aufweisen.

Befindet sich die radioaktive Quelle (4) im Strahlungswandler (2), so wird die von der radioaktiven Quelle (4) emittierte β-Strahlung vom Material des Strahlungswandlers (2) absorbiert und in weiche bis mittelharte Röntgenstrahlung umgewandelt, je nachdem, ob es sich bei dem Material des Strahlungswandlers (2) um Zink, Barium, Tellur (weiche Röntgenstrahlung) oder Silber (mittelharte Röntgenstrahlung) handelt. Die Röntgenquelle ist "angeschaltet".

Befindet sich die radioaktive Quelle (4) außerhalb des Strahlungswandlers (2) im Führungsrohr (3), so wird die emittierte β-Strahlung vollständig vom Material des Führungsrohres (3) absorbiert. Es erfolgt keine Umwandlung in Röntgenstrahlung. In diesem Fall ist die Röntgenquelle "abgeschaltet".

Das Führungsrohr (3), das aus einem körperverträglichen und β-Strahlung absorbierenden Material besteht, ist in einer bevorzugten Variante aus einer Metallegierung. Als Metallegierung kann ein Eisen und/oder Nickel enthaltender Stahl, eine Tantallegierung oder eine Nickel/Titan-Legierung eingesetzt werden. In einer besonders bevorzugten Variante wird als Eisen enthaltender Stahl der chirurgische Edelstahl CrNi316L eingesetzt.

Das an der radioaktiven Quelle (4) vorhandene Bewegungselement (5) ist in einer bevorzugten Ausführungsvariante ein Draht, ganz besonders bevorzugt aus einer Nickel/Titanlegierung.

Als Trägermaterial für das Nuklid der radioaktiven Quelle (4) kommen alle üblichen Materialien in Frage, die in der Lage sind, die erfindungsgemäß verwendeten Nuklide aufzunehmen. Erfindungsgemäß bevorzugt werden Zeolithe oder andere Kunststoffe als Träger eingesetzt, aber auch Keramiken wie Titandioxid, Zirkoniumdioxid, Aluminiumoxid oder Siliziumoxid kommen in Frage.

Die Herstellung der radioaktiven Quelle (4) erfolgt nach üblichen, dem Fachmann gut bekannten Methoden. So wird das erfindungsgemäß bevorzugte Promethium-147 beispielsweise in strahlenresistenten Kunststoff als Träger eingebettet und in einem dünnwandigen Titanrohr fixiert. Die Länge der radioaktiven Strahlenquelle (4) kann von 1 bis 70 mm betragen, vorzugsweise bis zu 30 mm.

Alle erfindungsgemäß eingesetzten Nuklide und Werkstoffe sind kommerziell erhältlich. Die Herstellung des Führungsrohres 3 mit Strahlunswandler 2 erfolgt durch Laserschweißen, gegebenenfalls durch Schweißvermittler. Andere Herstellungsmethoden wie Kleben kommen ebenfalls in Frage. Der Führungskopf 1 kann ebenfalls nach den genannten Technologien gefügt werden.

Für die Quellenherstellung sind verschiedene Techniken einsetzbar in Abhängigkeit vom Nuklid und seiner chemischen Form einerseits sowie von der Trägermatrix des Nuklides andererseits. In der bevorzugten Variante wird Pm-147 in strahlenresistente Zeolithe eingebracht und dann in einem dünnwandigen Rohr fixiert, das an einen Führungsdraht 5 geschweißt wird. Anstelle des Drahtes 5 kann auch ein geignetes Rohr 5 eingesetzt werden, an dessen Spitze die Zeolith-Kugeln oder Fäden fixiert sind.

In Abhängigkeit vom verwendeten Nuklid in der radioaktiven Quelle 4 kann die Lebensdauer der Röntgenquelle zwischen 55 Tagen (Sr-89) und 2,6 Jahren (Pm-147) betragen.

Durch die erfindungsgemäße Lösung sind nicht nur Röntgenquellen mit problemangepaßter Halbwertszeit, sondern auch mit problemangepaßtem Energiebereich auswählbar, je nachdem, aus welchem der o.g. Materialien der Strahlungswandler 2 besteht. Durch die erfindungsgemäße Lösung wird die Fremdstrahlung der Röntgenquelle äußerst gering gehalten, so daß eine gezielte und genau dosierte Strahlenbehandlung möglich wird. Die Dosisleistung der erfindungsgemäßen Röntgenquellen beträgt von 15 bis 20 Gray pro 3 min. im Abstand von 2 mm im Gewebe.

Gegenstand der Erfindung ist auch die Verwendung der erfindungsgemäßen Röntgenquelle zur intrakavitären oder intravaskulären Gewebebestrahlung, insbesondere zur Brachytherapie und Tumorbehandlung mittels Afterloadingtechnik. Die erfindungsgemäßen Röntgenquellen zur Tumorbestrahlung können per Afterloader appliziert und nach Positionierung "angeschaltet" werden. Dabei sind sowohl Gewebestrahlungen (Prostata, Brust, Gehirn, Lunge) als auch kavitäre Bestrahlungen möglich (Vagina, Rektum).

Figur 1 zeigt die erfindungsgemäße Röntgenquelle in einer vorteilhaften Ausgestaltung.

In Figur 1 bedeuten:
1 distaler Führungskopf
2 Strahlungswandler
3 Führungsrohr
4 radioaktive Quelle
5 Bewegungselement

## Patentansprüche

1. Radioaktive Röntgenquelle bestehend aus einer β-Strahlung emittierenden radioaktiven Strahlenquelle (4) und einem Strahlungswandler (2),
dadurch gekennzeichnet,
daß sie ein Führungsrohr (3) zum axialen Verschieben der darin angeordneten radioaktiven Strahlenquelle (4) mittels eines Bewegungselementes (5), das an der Strahlenquelle (4) vorhanden ist, aufweist,
daß der Teil des Führungsrohres (3), der an den distalen Führungskopf (1) des Führungsrohres (3) angrenzt, als Strahlungswandler (2) ausgestaltet ist
und daß der proximal an den Strahlungswandler (2) angrenzende Teil des Führungsrohres (3) aus einem die β-Strahlung absorbierenden Material besteht.

2. Röntgenquelle nach Anspruch 1,
dadurch gekennzeichnet, daß
das Führungsrohr (3) aus einem biologisch verträglichen Material besteht.

3. Röntgenquelle nach Anspruch 1 oder 2,
dadurch gekennzeichnet, daß
der Strahlungswandler (2) mindestens die Länge der radioaktiven Strahlenquelle (4) aufweist.

4. Röntgenquelle nach einem der Ansprüche 1 bis 3,
dadurch gekennzeichnet, daß
die Länge der radioaktiven Strahlenquelle (4) von 1 bis 70 mm beträgt, vorzugsweise bis zu 30 mm.

5. Röntgenquelle nach einem der Ansprüche 1 bis 4,
dadurch gekennzeichnet, daß
das Bewegungselement (5) an der radioaktiven Strahlenquelle (4) ein Draht ist.

6. Röntgenquelle nach einem der Ansprüche 1 bis 5,
dadurch gekennzeichnet, daß
das Nuklid der radioaktiven Quelle (4) Promethium (Pm)-147, Strontium (Sr)-89, Thallium (Tl)-204 oder Thulium (Tm)-170 ist, vorzugsweise Promethium (Pm)-147.

7. Röntgenquelle nach einem der Ansprüche 1 bis 6,
dadurch gekennzeichnet, daß
der Strahlungswandler (2) aus Silber, Zink, Barium, Tellur oder Wolfram besteht, vorzugsweise aus Silber.

8. Verwendung der Röntgenquelle nach einem der Ansprüche 1 bis 7 zur intrakavitären oder intravaskulären Gewebebestrahlung.

9. Verwendung nach Anspruch 8,
dadurch gekennzeichnet, daß
die Röntgenquelle in der Brachytherapie eingesetzt wird.

10. Verwendung nach Anspruch 8,
dadurch gekennzeichnet, daß
die Röntgenquelle zur Tumorbehandlung eingesetzt wird, vorzugsweise zur Afterloaderbehandlung von Tumoren.
